# EUROPÄISCHE PATENTANMELDUNG

(11) **EP 4 279 054 A1**
(43) Veröffentlichungstag der Anmeldung: **22.11.2023**
(21) Anmeldenummer: 22174670.4
(22) Anmeldetag: 20.05.2022
(51) Int. Cl.: A61J 1/06, A61M 5/28, A61M 5/24

(54) **WIRKSTOFFAMPULLE FÜR EINE MEDIZINISCHE SPRITZE UND VERFAHREN ZUM BEFÜLLEN EINER SOLCHEN WIRKSTOFFAMPULLE**

(71) Anmelder: Inductio AG, 4500 Solothurn (CH)
(72) Erfinder: KOLLER, Horst, 8730 Uznach (CH)
(74) Vertreter: Tergau & Walkenhorst Intellectual Property GmbH

(57) **Zusammenfassung**

Eine Wirkstoffampulle (2) für eine medizinische Spritze (1) mit einem mit einem Wirkstoff befüllbaren, im Wesentlichen zylindrischen Hohlkörper (16) aus einem ersten Kunststoffmaterial soll die Abfüllung des Wirkstoffs erleichtern und auch hohe Durchsatzraten bei der Abfüllung in automatisierten Prozessen ermöglichen. Dazu ist erfindungsgemäß der Hohlkörper (16) an einem Ende (4) mit einem integrierten Verschlusskörper (50) aus einem zweiten, im Vergleich zum ersten weicheren Kunststoffmaterial versehen, wobei der von Hohlkörper (16) und Verschlusskörper (50) gebildete Hybridkörper erhalten ist durch ein 2K-Spritzgussverfahren.

## Beschreibung

Die Erfindung betrifft eine Wirkstoffampulle für eine medizinische Spritze, insbesondere zur Verwendung in der Lokalanästhesie, beispielsweise bei zahnärztlichen Behandlungen. Sie bezieht sich weiter auf ein Verfahren zum Befüllen einer solchen Wirkstoffampulle mit Wirkstoff.

In vielfältigen Anwendungsbereichen, insbesondere auch bei der Lokalanästhesie bei der zahnärztlichen Behandlung, werden in großer Zahl medizinische Spritzen mit mit dem vorgesehenen Wirkstoff vorbefüllten Wirkstoffampullen verwendet. Bei einer solchen Spritze, wie sie beispielsweise aus der EP 3 738 629 A1 bekannt ist, wird die vorbefüllte Wirkstoffampulle, auch als Kartusche oder Patrone bezeichnet, einerseits mit einem Griffstück oder Rahmen und andererseits mit einem Nadelkopf oder Nadelträger versehen, so dass die Spritze einsatzbereit wird. Der Nadelkopf oder Nadelträger umfasst dabei die eigentliche Spritzennadel, deren proximales Ende bei der Anbringung des Nadelkopfs an der Wirkstoffampulle deren endseitige Versiegelung durchstößt und somit medienseitig mit dem Innenraum der Ampulle verbunden wird. Das Griffstück umfasst hingegen üblicherweise einen in seiner Längsrichtung verschiebbaren Betätigungsstößel für den in der Ampulle angeordneten Spritzenkolben oder Stopfen, über den der Wirkstoff in Richtung der Nadel gedrückt und über diese ausgegeben werden kann.

Die Nutzung solcher vorbefüllter Wirkstoffampullen ermöglicht auf Seiten des Verwenders, also beispielsweise des Zahnarztes, eine deutlich vereinfachte Handhabung, da keine vergleichsweise aufwendige Abfüllung des Wirkstoffs beim Verwender erforderlich ist. Zudem können solche Wirkstoffampullen maschinell und automatisiert zentral und in hohen Stückzahlen und mit hohen Taktraten befüllt werden. Dennoch besteht der Bedarf, die Handhabung solcher Systeme und auch die Abfüllung als solche noch einfacher und damit effizienter zu gestalten.

Der Erfindung liegt nunmehr die Aufgabe zugrunde, eine Wirkstoffampulle für eine Spritze der oben genannten Art anzugeben, mit der diesem Bedürfnis Rechnung getragen ist. Des Weiteren soll ein besonders günstiges Verfahren zur Befüllung einer solchen Wirkstoffampulle angegeben werden.

Bezüglich der Wirkstoffampulle wird diese Aufgabe erfindungsgemäß gelöst mit einem mit einem Wirkstoff befüllbaren, im Wesentlichen zylindrischen Hohlkörper aus einem ersten Kunststoffmaterial, der an einem Ende mit einem integrierten Verschlusskörper aus einem zweiten, im Vergleich zum ersten weicheren Kunststoffmaterial versehen ist, erhalten durch ein 2K-Spritzgussverfahren.

Die Erfindung geht von der Überlegung aus, dass für eine besonders effiziente und ressourcenschonende Herstellung und auch Befüllung der Ampulle die Anzahl der verwendeten Komponenten oder Bauteile und auch die Arbeitsschritte für deren Montage möglichst geringgehalten werden sollen. Um dies zu ermöglichen, ist vorgesehen, den zylindrischen Hohlkörper von vornherein gemeinsam und in einem einzigen Arbeitsschritt mit dem Verschlusskörper herzustellen. Dazu ist ein 2K-Spritzgussverfahren vorgesehen, wobei der solchermaßen hergestellte "2K-Hybridkörper" auch unmittelbar nach seiner Herstellung der Befüllung zugeführt werden kann.

Funktional bedingt sind die Kunststoffe für den Hohlkörper einerseits und den Verschlusskörper andererseits vorteilhafterweise geeignet gewählt. Der Hohlkörper oder Ampullenkörper kann als Kunststoffteil aus Cyclo-Olefin-Copolymer (COC), PC oder PP gefertigt, ist in ganz besonders bevorzugter Ausgestaltung aber als Kunststoffteil aus Cyclo-Olefin-Polymer (COP) hergestellt. Diese Werkstoffe zeichnen sich durch hohe Bruchfestigkeit und glasähnliche Transparenz aus. Sie setzen zudem keine Alkali-Ionen frei, so dass das Risiko einer pH-Wert-Verschiebung im vorgehaltenen Wirkstoff ausgeschlossen ist. Damit wird dieser Werkstoff erfindungsgemäß als sogar für die Primärverpackung auch anspruchsvoller Medikamente, insbesondere für sensible biotechnologisch hergestellte Wirkstoffe, geeignet angesehen und verwendet. Zudem ist dieser Werkstoff für die vorgesehene Fertigung im Spritzgießverfahren und somit für besonders präzise Dimensionierung besonders geeignet.

Der Verschlusskörper soll demgegenüber die Möglichkeit bieten, dass zur Entnahme des Wirkstoffs, also zur Verabreichung des Wirkstoffs bei Betätigung der Spritze, eine Spritzennadel problemlos hindurchgestochen werden kann. Dazu bildet der Verschlusskörper vorteilhafterweise in einem frontalen Zentralbereich eine von einer Hohlnadel durchstechbare Membran aus. In vorteilhafter Weiterbildung ist an diese Membran umlaufend eine Fixierschürze angeformt, die an der Innenwand des Hohlkörpers dichtend anliegt.

Die gemäß einem Aspekt der Erfindung vorgesehene Herstellung des Hybridkörpers aus Hohlkörper der Wirkstoffampulle mit integriertem Verschlusskörper in einem einzigen Arbeitsschritt im 2K-Spritzgussverfahren kann auf besonders vorteilhafte Weise auch gezielt genutzt werden, um die Position des Verschlusskörpers im Hohlkörper durch Formschluss bzw. Hinterschneidungen zu fixieren. Dazu weist der Hohlkörper vorteilhafterweise in seinem den Verschlusskörper enthaltenden Endbereich eine innenseitig umlaufende Wulst auf, die in eine korrespondierende umlaufende Außennut in der Fixierschürze eingreift.

Gemäß einem Aspekt der Erfindung ist die Wirkstoffampulle der beschriebenen Art Teil einer medizinischen Spritze, wobei an der Wirkstoffampulle endseitig einerseits ein eine Hohlnadel tragender Nadelkopf und andererseits ein Griffstück anbringbar sind.

In einer als eigenständig und unabhängig erfinderisch angesehenen Ausführung ist eine medizinische Spritze, insbesondere der vorstehend beschriebenen Art, mit einem Nadelschutz ausgestattet. Zur Vermeidung oder Verringerung von Kontaminations- oder Verletzungsrisiken nach dem Gebrauch von Medikamentenspritzen und insbesondere zur Vermeidung des mehrfachen Gebrauchs von Spritzennadeln durch verschiedene Nutzer finden nämlich Spritzen mit Nadelschutzsystemen zunehmend Verwendung. Ein solcher Nadelschutz kann beispielsweise in der Art eines Rückzieh- oder Retraktionssystems ausgestaltet sein, bei dem die Spritzennadel nach Abgabe des in der Spritze vorgehaltenen Wirkstoffs in den Spritzenkörper eingezogen und von diesem vollständig umschlossen wird. Ein Zugang zur Spritze und damit ein Verletzungsrisiko, oder auch das Risiko mehrfachen Gebrauchs derselben Nadel, kann damit weitgehend ausgeschlossen werden.

Vorliegend ist in als eigenständig erfinderisch angesehener Ausgestaltung ein solcher Nadelschutz realisiert, indem der Nadelkopf einen Grundkörper aufweist, an dem zur Bildung einer Nadelschutzhülle über ein Gelenk ein Schutzkörper schwenkbar angebracht ist, der in seiner Länge die freie Länge der Hohlnadel übertrifft und diese somit endseitig überragt.

Gemäß einem Aspekt der Erfindung wird die Wirkstoffampulle der vorstehend beschriebenen Art hergestellt, indem der Hohlkörper und der in diesem vorgesehene Verschlusskörper in einem gemeinsamen Arbeitsschritt in einem 2K-Spritzgussverfahren hergestellt werden.

Bezüglich des Verfahrens zum Befüllen einer Wirkstoffampulle der vorstehend beschriebenen Art mit Wirkstoff wird die genannte Aufgabe gemäß einem Aspekt der Erfindung gelöst, indem der Hohlkörper mit seiner Längsrichtung in eine vertikale Position gebracht wird, wobei das mit dem Verschlusskörper versehene Ende abwärts ausgerichtet wird und der Hohlkörper anschließend über sein offenes Ende befüllt wird. Anschließend kann in dieses offene Ende dann ein geeigneter Spritzenstopfen eingeführt werden, so dass der eingefüllte Wirkstoff dicht im Hohlkörper eingeschlossen wird.

Ein Ausführungsbeispiel der Erfindung wird anhand einer Zeichnung näher erläutert. Darin zeigen:
- Fig. 1: eine medizinische Spritze, insbesondere zur Verwendung in der Lokalanästhesie, beispielsweise bei zahnärztlichen Behandlungen, in perspektivischer Ansicht,
- Fig. 2: die Spritze nach Fig. 1 mit entfernter Nadelschutzkappe in perspektivischer Ansicht,
- Fig. 3: die Spritze nach Fig. 1 im Längsschnitt,
- Fig. 4: die Spritze nach Fig. 2 im Längsschnitt,
- Fig. 5: den Nadelkopf der Spritze gemäß Fig. 1 in perspektivischer Ansicht,
- Fig. 6: den Nadelkopf der Spritze gemäß Fig. 1 in seitlicher Ansicht,
- Fig. 7: den Nadelkopf der Spritze gemäß Fig. 1 im Längsschnitt,
- Fig. 8: den Nadelkopf der Spritze gemäß Fig. 1 in einsatzbereitem Zustand in seitlicher Ansicht,
- Fig. 9: den Nadelkopf gem. Fig. 8 im Längsschnitt,
- Fig. 10: eine ausschnittsweise Vergrößerung von Fig. 9,
- Fig. 11: eine Sequenz beim Abschwenken eines Schutzkörpers von der Spritzennadel,
- Fig. 12: ein Griffstück der Spritze gem. Fig. 1 in perspektivischer Ansicht,
- Fig. 13: das Griffstück gem. Fig. 12 im Längsschnitt,
- Fig. 14: ein zur Entsorgung vorgesehenes Ensemble aus entleerter Wirkstoffampulle und Nadelkopf mit abgeknickter Nadel,
- Fig. 15: die Wirkstoffampulle der Spritze nach Fig. 1 in perspektivischer Ansicht,
- Fig. 16: die Wirkstoffampulle nach Fig. 15 in seitlicher Ansicht, und
- Fig. 17: die Wirkstoffampulle nach Fig. 15 im Längsschnitt.

Gleiche Teile sind in allen Figuren mit denselben Bezugszeichen versehen.

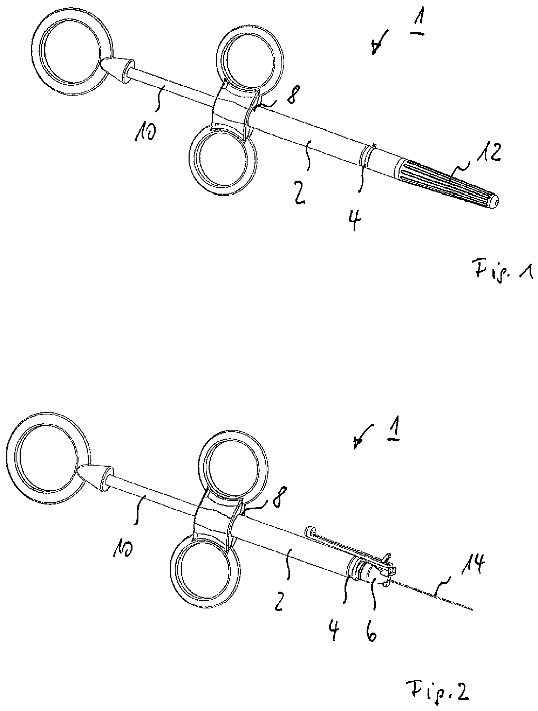

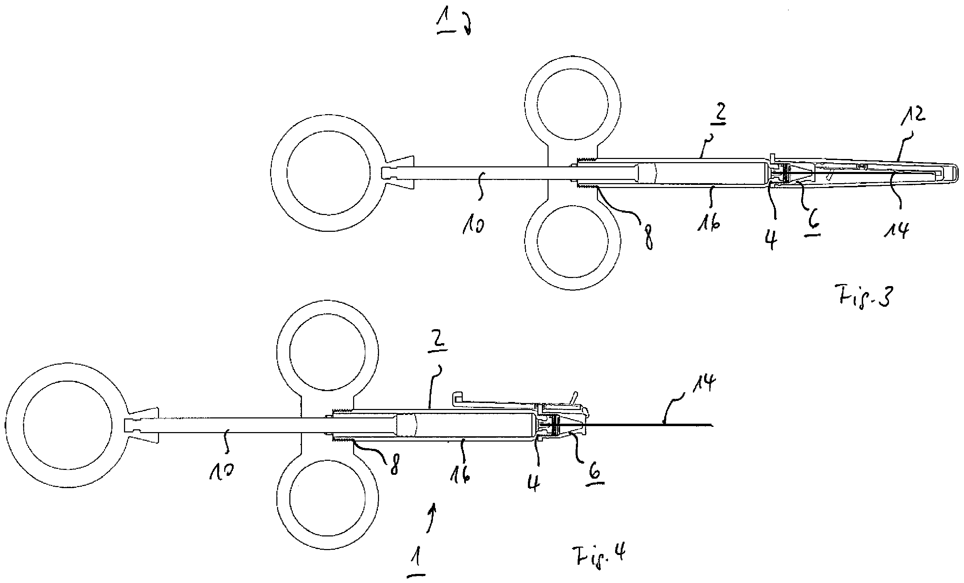

Die medizinische Spritze 1 gemäß Fig. 1 umfasst im Wesentlichen drei Baugruppen, nämlich eine Wirkstoffampulle 2 (auch als Kartuschen- oder Patroneneinheit bezeichnet), die einerseits an ihrem distalen Ende 4 mit einem Nadelkopf 6 oder Nadelhalter und andererseits an ihrem proximalen Ende 8 mit einem Griffstück 10 verbunden ist. In Fig. 1 ist die Spritze 1 zudem mit am Nadelkopf 6 angebrachter Nadelschutzkappe 12 gezeigt. Fig. 2 zeigt die Spritze 1 hingegen in einsatzfähigem Zustand mit entfernter Nadelschutzkappe 12, so dass die im Nadelkopf 6 angebrachte Hohlnadel 14 freiliegt. In den Fig. 3 und 4 ist die Spritze 1 gem. Fig. 1 bzw. gem. Fig. 2 jeweils im Längsschnitt gezeigt.

Die Wirkstoffampulle 2 umfasst einen entsprechend einer herkömmlichen Bauweise zylindrisch oder rohrförmig ausgeführten, ein Spritzengehäuse bildenden Hohlkörper 16, der zur Aufnahme des medizinischen Wirkstoffs vorgesehen ist. Die Wirkstoffampulle 2 ist dabei als vorbefüllte Wirkstoffampulle ausgestaltet, die in großer Stückzahl an einem zentralen Herstellungsort mit dem vorgesehenen Wirkstoff befüllt und anschließend an den Verwender, beispielsweise den Zahnarzt, geliefert wird. Um die Spritze 1 dann einsatzfertig zu machen, muss der Verwender den Nadelkopf 6 einerseits und das Griffstück 10 andererseits an dem Hohlkörper 16 anbringen.

Der Nadelkopf 6 oder Nadelhalter kann dabei am vorderen oder distalen Ende 4 des Hohlkörpers 16 angeschraubt oder auch aufgesteckt werden, je nachdem, welche Bauweise für die Verbindung gewählt ist. Der Nadelkopf 6, der in diesem Moment der Anbringung noch mit der Nadelschutzkappe 12 versehen ist, weist dabei eine an sich als eigenständig erfinderisch angesehene Ausgestaltung auf. Dabei ist dem Umstand Rechnung getragen, dass es ein zunehmendes Bedürfnis und teilweise auch schon in rechtliche oder regulatorische Vorschriften eingeflossen ist, medizinische Spritzen mit so genannten Nadelschutzsystemen auszurüsten. Zunehmende gesetzliche Vorgaben zur sicheren Arbeitsumgebung in verschiedenen Ländern verpflichten nämlich beispielsweise die Arbeitgeber im medizinischen Bereich zum Schutz ihrer Mitarbeit er vor Nadelstichverletzungen. Dazu kann dann vorgesehen sein, beispielsweise ein System aus Schutzkappen vorzusehen, die über die Nadel gesteckt werden, wenn diese nicht in Benutzung ist, oder es können so genannten Retraktionssysteme vorgesehen sein, bei denen die Nadel nach Gebrauch in das zwischenzeitlich vom Wirkstoff entleerte Spritzengehäuse zurückgezogen wird, so dass die Nadelspitze nicht mehr freiliegt.

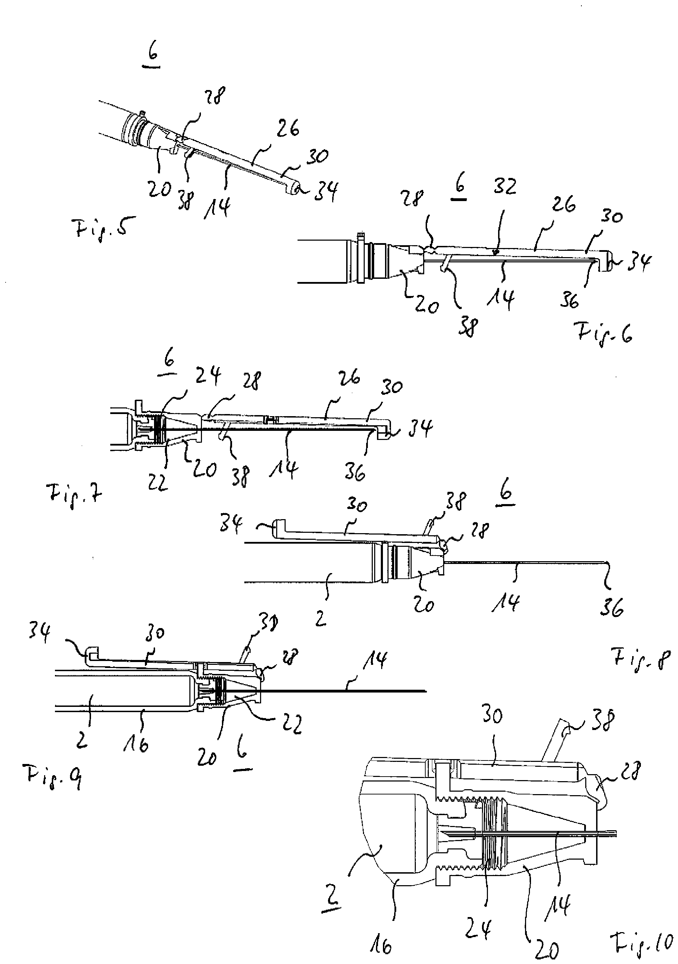

Bei der vorliegenden, als eigenständig erfinderisch angesehenen Ausführungsform ist der an den Hohlkörper 16 anbringbare Nadelkopf 6 oder Nadelhalter mit einem integrierten Nadelschutzsystem versehen. Der Nadelkopf 6 - nach Entfernung der Nadelschutzkappe 12 - ist in perspektivischer Ansicht in Fig. 5, in seitlicher Ansicht in Fig. 6 und im Längsschnitt in Fig. 7 jeweils vergrößert gezeigt.

Wie diesen Darstellungen gut entnehmbar ist, umfasst der Nadelkopf 6 einen den eigentlichen Nadelhalter bildenden Grundkörper 20, vorzugsweise gefertigt aus einem harten Kunststoff wie beispielsweise PP, POM oder PC. Der Grundkörper 20 lagert und trägt dabei die Hohlnadel 14; er ist innenseitig mit einem Aufnahmekanal 22 versehen, in den das distale Ende 4 des Hohlkörpers 16 eingebracht werden kann. Gezeigt ist vorliegend die Variante, bei der der Nadelkopf 6 auf das distale Ende 4 des Hohlkörpers 16 aufgeschraubt werden soll, und demzufolge weist im gezeigten Ausführungsbeispiel der Grundkörper 20 in seinem Aufnahmekanal 22 ein Innengewinde 24, vorzugsweise ein Fein-Innengewinde, auf.

Zur Bereitstellung des gewünschten Nadelschutzes ist am Grundkörper 20 eine Nadelschutzhülle 26 über ein Gelenk 28 schwenkbar angebracht. Die Nadelschutzhülle 26 wird dabei von einem länglich ausgedehnten Schutzkörper 30 gebildet, der in seiner Länge die freie Länge der Hohlnadel 14 übertrifft und diese somit endseitig überragt. Des Weiteren ist die Breite des Schutzkörpers 30 derart gewählt, dass er die Hohlnadel 14 beidseitig nach außen hin deutlich überragt. Der Schutzkörper 30 kann zudem innenseitig eine Aufnahmerinne 32 aufweisen, in die die Hohlnadel 14 in der in den Fig. 5 - 7 gezeigten Position des Schutzkörpers 30 eingebettet sein kann. Endseitig ist am Schutzkörper 30 eine Deckplatte 34 angeformt, die in der dort gezeigten Position das freie Ende 36 der Hohlnadel 14 abdeckt. In der in den Fig. 5 - 7 gezeigten Position ist das freie Ende 36 der Hohlnadel 14 somit vollständig abgeschirmt, und kein Kontakt und somit auch keine Verletzung durch die Hohlnadel 14 ist damit möglich.

In dieser Position befindet sich das System, wenn die äußere Schutzkappe 12 abgenommen wird. Da die Hohlnadel 14 in diesem Zustand noch von dem Schutzkörper 30 abgeschirmt wird, ist noch kein Einstechen der Nadel 14 möglich. Um diese einsetzbar zu machen, wird daher mittels eines am Schutzkörper 30 angeformten Betätigungselements 38 um das Gelenk 28 herum von der Hohlnadel 14 weggeschwenkt, so dass diese nunmehr freigelegt und freigegeben wird. In diesem Zustand, in dem die Spritze 1 nun zur Abgabe des Wirkstoffs bereit ist, ist der Nadelkopf 6 in Fig. 8 in seitlicher Ansicht, in Fig. 9 im Längsschnitt und in Fig. 10 im Längsschnitt vergrößert gezeigt.

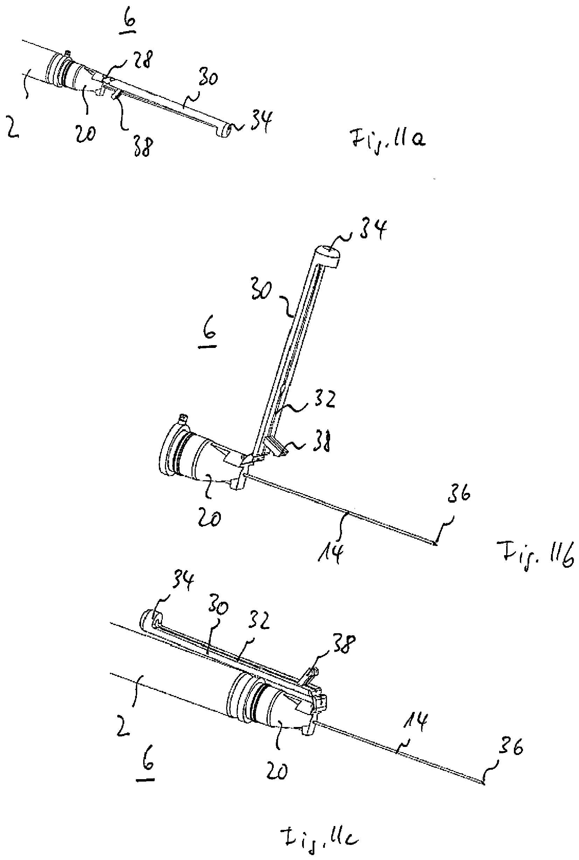

Das Abschwenken des Schutzkörpers 30 von der Hohlnadel 14, mit dem diese freigelegt und somit zur Benutzung bereitgestellt wird, ist zur Verdeutlichung noch einmal in der Sequenz nach Fig. 11 gezeigt, und zwar ausgehend von der Ausgangsposition direkt nach Abnehmen der äußeren Nadelschutzkappe 12 (Fig. 11a) über eine Zwischenposition mit halb abgeschwenktem Schutzkörper 30 (Fig. 11 b) bis hin zur Endposition (Fig. 11c), in der der Schutzkörper 30 vollständig von der Nadel 14 abgeschwenkt ist und rückseitig an der Außenseite des Hohlkörpers 16 anliegt.

Nach Gebrauch der Spritze 1 kann der Schutzkörper 30 um das Gelenk 28 herum wieder zurück geschwenkt werden, so dass er die Hohlnadel 14 erneut wie vorstehend beschrieben abschirmt und damit Verletzungen durch Berührungen der Nadel 14 sicher vermieden sind. Um aber die Verletzungsgefahr an der dann benutzten Nadel 14 noch weiter zu vermindern, ist gemäß einem weiteren Aspekt der Erfindung vorgesehen, den Schutzkörper 30 um das Gelenk 28 herum noch weiter zu verschwenken, bis die Nadel 14 in ihrem Nadellager im Grundkörper 20 abbricht. Damit wird die Nadel 14 nunmehr endgültig in ihrer Position innerhalb des Abschirmbereichs des Schutzkörpers 30 fixiert, und zudem wird damit unmittelbar deutlich, dass die Spritze 1 bereits benutzt war und die (nunmehr verbrauchte) Wirkstoffampulle 2 gemeinsam mit der noch daran montierten, nunmehr zerbrochenen Nadel 14 entsorgt werden soll. Damit sind Irrtümer und Verwechslungen, die beispielsweise zu einem versehentlichen Wiederbenutzen der Spritze 1 führen könnten, sicher ausgeschlossen.

Die Anbringung der genannten Komponenten des Nadelschutzes 26 an der Hohlnadel 14 kann in der Art eines "Overmolding" in einem einzigen, gemeinsamen Arbeitsschritt erfolgen.

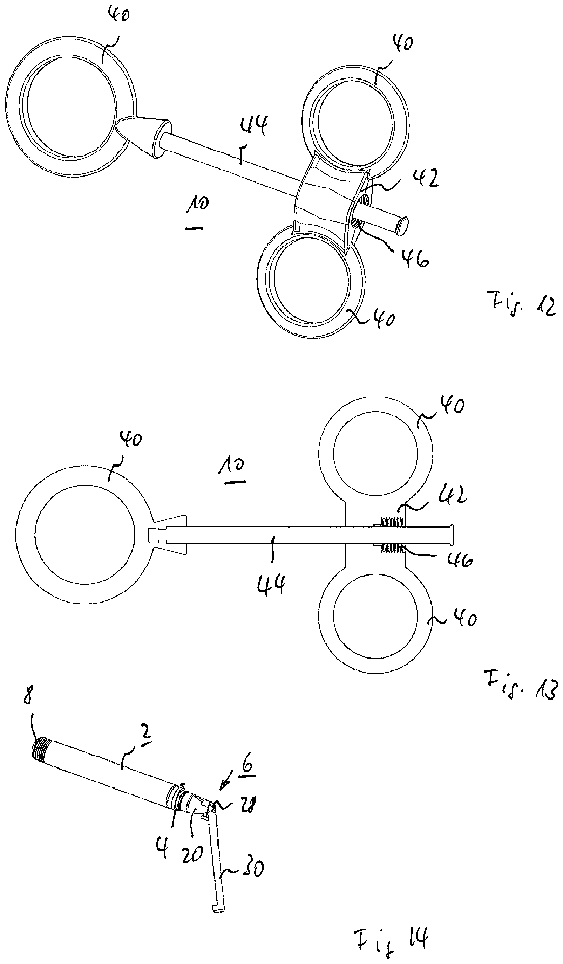

Das Griffstück 10 muss wie vorstehend beschrieben vor dem Einsatz der Spritze 1 ebenfalls zunächst am Hohlkörper 16 angebracht werden. Es umfasst, wie dies aus der perspektivischen Darstellung in Fig. 12 und der Darstellung im Längsschnitt in Fig. 13 ersichtlich ist, ein mit Fingerringen 40 versehenes Basiselement 42, in dem ein endseitig ebenfalls mit einer Fingerlasche 40 versehener Betätigungsstößel 44 in seiner Längsrichtung verschiebbar geführt ist. Zur Verbindung mit der Wirkstoffampulle 2 ist auch hier im Ausführungsbeispiel eine Schraubverbindung vorgesehen, und dementsprechend ist das Basiselement 42 mit einem Innengewinde 46 versehen, in das das proximale Ende 8 der Wirkstoffampulle 2 eingeschraubt werden kann. Im Hinblick auf an sich übliche Auslegungsziele einer an sich einfach gehaltenen, aber hochwertigen und insbesondere hohen Sicherheitserfordernissen genügenden Bauweise sind die genannten Komponenten des Griffstücks 10 im Ausführungsbeispiel aus Polyphenylsulfon (PPSU) ausgeführt, einem hochtechnischen Spezialkunststoff, der bei hoher mechanischer Stabilität und Belastbarkeit nahezu beliebig oft sterilisierbar ist und bei medizinischen Produkten als Metallersatz gilt. In seiner Gesamtheit ist das Griffstück 10 somit, insbesondere aufgrund seiner Materialwahl, vielfach und wiederholt sterilisierbar und somit vielfach wiederverwendbar.

Nach Beendigung der Wirkstoffabgabe in der oben beschriebenen Weise kann dann die - nunmehr entleerte - Wirkstoffampulle 2 wieder vom Griffstück 10 getrennt werden, indem die Schraubverbindung zwischen dem Hohlkörper 16 und dem Griffstück 10 wieder gelöst wird. Gemeinsam mit dem Nadelkopf 6 und der daran befindlichen, zwischenzeitlich gemeinsam mit dem Schutzkörper 30 abgeknickten Hohlnadel 14 kann das Ensemble aus Nadelkopf 6 und - entleerter - Wirkstoffampulle 2 einer Entsorgung zugeführt werden. Dieses Ensemble ist in perspektivischer Ansicht in Fig. 14 gezeigt.

Durch die vorstehend beschriebene Bauweise und Auslegung sind für medizinisches Personal und Patienten insbesondere folgende Vorteile erreichbar:
- sehr einfache Handhabung
- extrem geringes Gewicht
- kontrollierte Systemaktivierung mit nur einer Hand
- irreversibler und deutlich erkennbarer Einrasteffekt
- unveränderte Injektionstechnik
- normale Entsorgung und geringer Abfall; durch das Nadelschutzkonzept handelt es sich nicht um Sondermüll
- kein zusätzlich benötigter Platzbedarf
- Nadel und Spritzeninhalt sind komplett sichtbar

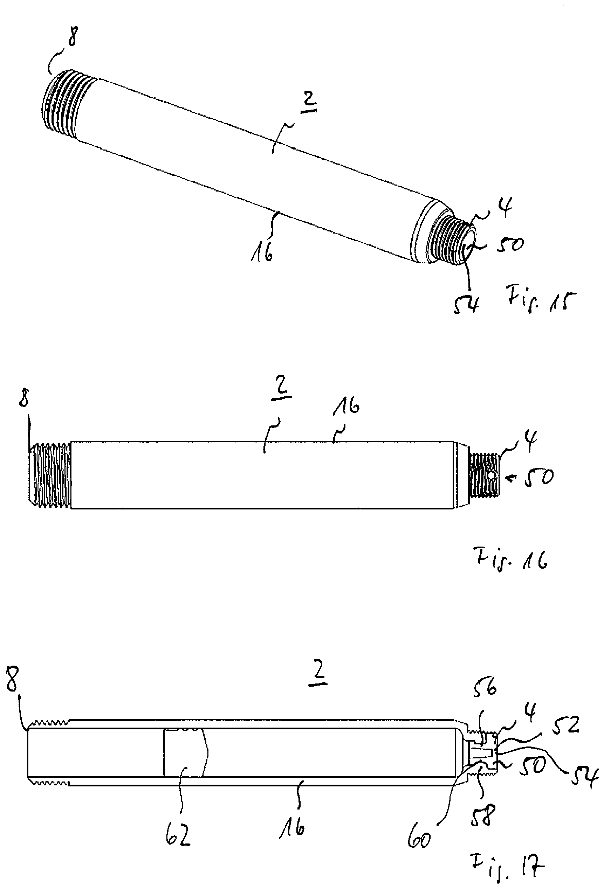

Gemäß einem weiteren, als unabhängig und eigenständig erfinderisch angesehenen Aspekt der Erfindung ist die Wirkstoffampulle 2 gezielt für einen hoch effizienten, für große Stückzahlen geeigneten Herstell- und Abfüllprozess geeignet ausgeführt. Dazu ist die Wirkstoffampulle 2, die in Fig. 15 in perspektivischer Ansicht, in Fig. 16 in seitlicher Ansicht und in Fig. 17 im Längsschnitt gezeigt ist, als Hohlkörper 16 ausgeführt, der an seinem distalen, zur Verbindung mit dem Nadelkopf 6 vorgesehenen Ende 4 mit einem integrierten, vom proximalen Ende der Hohlnadel 14 durchstechbaren Verschlusskörper 50 versehen. Der Verschlusskörper 50 ist dabei, wie insbesondere der Darstellung im Längsschnitt in Fig. 17 entnehmbar ist, als dreidimensionaler Formkörper ausgeführt. Dieser bildet in einem frontalen Zentralbereich 52 eine vergleichsweise dünn ausgeführte Membran 54 aus TPE aus, die bei der Anbringung des Nadelkopfs 6 vom proximalen Ende der Hohlnadel 14 durchstochen werden kann.

An die Membran 54 ist umlaufend eine Fixierschürze 56 angeformt, die an der Innenwand des Hohlkörpers 16 dichtend anliegt und den Verschlusskörper 50 in seiner Position im Hohlkörper 16 fixiert. Um dabei gerade in Längsrichtung des Systems ein unbeabsichtigtes Verschieben des Verschlusskörpers 50 relativ zum Hohlkörper, beispielsweise ein Herausrutschen, sicher zu vermeiden, weist der Hohlkörper 16 in seinem distalen Endbereich eine innenseitig umlaufende Wulst 58 auf, die in eine korrespondierende umlaufende Außennut 60 in der Fixierschürze 56 eingreift. Damit ist die Position des Verschlusskörpers 50 im Hohlkörper 16 in Längsrichtung festgelegt.

Hinsichtlich der als eigenständig erfinderisch angesehenen Materialwahl für das Spritzengehäuse bzw. den dieses bildenden Hohlkörper 16 ist insbesondere hohen Ansprüchen an die zuverlässige vorübergehende Lagerung des medizinischen Wirkstoffs einhergehend mit einer besonders hohen Sicherheit im Umgang mit den Komponenten Rechnung getragen. Der als zylindrischer Hohlkörper ausgeführte Spritzenkörper 16 kann dabei aus COC, PC oder PP gefertigt sein, ist im gezeigten Ausführungsbeispiel aber in als erfinderisch angesehener Ausgestaltung aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist.

Der Verschlusskörper 50 ist demgegenüber aus dem vergleichsweise weichen TPE gefertigt, so dass bei hoher Dichtigkeit auch das vorgesehene Durchstechen der Hohlnadel 14 problemlos möglich ist.

In als ebenfalls eigenständig erfinderisch angesehener Ausgestaltung ist für die Herstellung des mit dem Verschlusskörper 50 versehenen Hohlkörpers 16 ein 2K-Spritzgussverfahren vorgesehen. Dabei werden die Komponenten in einem einzigen Prozess gemeinsam hergestellt, so dass die vorgesehene verschiebungssichere Positionierung des Verschlusskörpers 50 im Hohlkörper 16 problemlos und auf einfache Weise erreicht werden kann. Der Spritzenkörper 6 ist vorzugsweise im Spritzgießverfahren gefertigt, wobei unter anderem die Formgebung derart erfolgt, dass mögliche Totvolumina im Inneren besonders geringgehalten sind.

Korrespondierend zum Verschlusskörper 50 ist ein innerhalb des Hohlkörpers 16 verschiebbarer Stopfen 62, ebenfalls hergestellt aus TPE, vorgesehen. Dieser ist derart dimensioniert, dass er dichtend an der Innenwand des Hohlkörpers 16 anliegt; im Längsbereich des Innenraums des Hohlkörpers 16 zwischen dem Verschlusskörper 50 und dem Stopfen 62 wird somit das Reservoir für den Wirkstoff ausgebildet, in dem dieser vorgehalten werden kann. Bei angebrachtem Nadelkopf 6, bei dem die Hohlnadel 14 durch die Membran 54 in dieses Reservoirvolumen hineinragt, kann der Stopfen 62 in Richtung zum distalen Ende 4 und somit zum Verschlusskörper 50 innerhalb des Hohlkörpers 16 verschoben werden, so dass der Wirkstoff durch die Hohlnadel 14 aus dem Innenraum herausgedrückt wird. Beim Einsatz der Spritze 1 und montiertem Griffstück 10 schlägt der Betätigungsstößel 44 mit seinem freien Ende an den Stopfen 62 an, so dass dieser mittels des Betätigungsstößels verschoben werden kann.

Die Wirkstoffampulle 2 ist im Ausführungsbeispiel für eine Schraubverbindung sowohl mit dem Nadelkopf 6 als auch mit dem Griffstück 10 ausgelegt und daher am distalen und am proximalen Ende 4, 8 jeweils mit einem entsprechenden Schraubgewinde versehen. Alternativ könnte aber auch eine oder beide dieser Verbindungen als Press- oder Steckverbindung und dementsprechend ohne Gewinde ausgeführt sein.

Gemäß einem Aspekt der Erfindung ist die Wirkstoffampulle 2 in ihren Außenabmessungen identisch zu den bisher verwendeten Glasampullen ausgeführt. Damit ist eine Rückwärtskompatibilität zu bestehenden Systemen und die Kombinierbarkeit mit vorhandenen Komponenten wie beispielsweise Griffstücken gegeben.

Die beschriebene Bauweise der Wirkstoffampulle 2 erlaubt ein ebenfalls als eigenständig erfinderisch angesehenes revolutionäres, neuartiges Abfüllverfahren, das auf besonders einfache Weise besonders hohe Durchsatzraten und damit Stückzahlen bei der Abfüllung ermöglicht. Dabei wird zunächst in einem ersten Schritt, wie vorstehend beschrieben, der mit dem Verschlusskörper 50 endseitig verschlossene Hohlkörper 16 als 2K- oder Hypridkomponente hergestellt. Weiterhin wird der Stopfen 62 bereitgestellt. Der Hybridkörper aus Hohlkörper 16 und integriertem Verschlusskörper 50 wird dabei gemäß einem Aspekt der Erfindung unter Einhaltung der Vorgaben und Vorschriften für Primärpackmittel hergestellt, so dass er direkt, ohne Umwege und ohne weitere Zwischenschritte von der Spritzgussanlage zur Abfüll-Anlage überführt werden kann. Die sonst in diesem Zusammenhang notwendigen Zwischenschritte Waschen, Silikonisieren oder Sterilisieren sind damit nicht mehr notwendig, was eine erhebliche Prozessvereinfachung bedeutet.

Bei der Abfüllung, also in der Abfüllanlage, wird der mit dem Verschlusskörper 50 versehene Hohlkörper 16 gemäß einem weiteren Aspekt der Erfindung sodann vertikal, also mit seiner Längsachse parallel zur Erdanziehungskraft, ausgerichtet, und zwar derart, dass das in diesem Zustand noch offene proximale Ende 8 nach "oben" und das mit dem Verschlusskörper 50 verschlossene distale Ende 4 nach "unten", also zur Erde hin, weist. Damit kann die Befüllung der Wirkstoffampulle 2 mit dem Wirkstoff beginnen. Dies erfolgt gemäß einem Aspekt der Erfindung in einer Groß-Abfüllanlage, in der eine Vielzahl von Ampullen parallel und gleichzeitig befüllt werden können. Nach Einbringung der vorgesehenen Wirkstoffmenge in den Hohlkörper 16 wird schließlich der Stopfen 62 in das proximale Ende 8 des Hohlkörpers 16 eingeschoben und verschließt somit den mit dem Wirkstoff befüllten Innenraum. Anschließend erfolgt in herkömmlicher Weise das Versiegeln und die Endkontrolle, und die befüllte Wirkstoffampulle 2 ist versandfertig.

### Bezugszeichenliste

- 1: Medizinische Spritze
- 2: Wirkstoffampulle
- 4: distales Ende
- 6: Nadelkopf
- 8: proximales Ende
- 10: Griffstück
- 12: Nadelschutzkappe
- 14: Hohlnadel
- 16: Hohlkörper
- 20: Grundkörper
- 22: Aufnahmekanal
- 24: Innengewinde
- 26: Nadelschutzhülle
- 28: Gelenk
- 30: Schutzkörper
- 32: Aufnahmerinne
- 34: Deckplatte
- 36: freies Ende
- 38: Betätigungselement
- 40: Fingerring
- 42: Basiselement
- 44: Betätigungsstößel
- 46: Innengewinde
- 50: Verschlusskörper
- 52: frontaler Zentralbereich
- 54: Membran
- 56: Fixierschürze
- 58: Wulst
- 60: Außennut
- 62: Stopfen

## Patentansprüche

1. Wirkstoffampulle (2) für eine medizinische Spritze (1) mit einem mit einem Wirkstoff befüllbaren, im Wesentlichen zylindrischen Hohlkörper (16) aus einem ersten Kunststoffmaterial, der an einem Ende (4) mit einem integrierten Verschlusskörper (50) aus einem zweiten, im Vergleich zum ersten weicheren Kunststoffmaterial versehen ist, erhalten durch ein 2K-Spritzgussverfahren.

2. Wirkstoffampulle (2) nach Anspruch 1, deren Hohlkörper (16) als Kunststoffteil aus Cyclo-Olefin-Copolymer (COC), PC oder PP, bevorzugt aus Cyclo-Olefin-Polymer (COP), gefertigt ist.

3. Wirkstoffampulle (2) nach Anspruch 1 oder 2, deren Verschlusskörper (50) in einem frontalen Zentralbereich (52) eine von einer Hohlnadel (14) durchstechbare Membran (54) ausbildet.

4. Wirkstoffampulle (2) nach Anspruch 3, deren Verschlusskörper (50) eine an die Membran (54) umlaufend angeformte Fixierschürze (56) aufweist, die an der Innenwand des Hohlkörpers (16) dichtend anliegt.

5. Wirkstoffampulle (2) nach Anspruch 4, deren Hohlkörper (16) in seinem den Verschlusskörper (50) enthaltenden Endbereich eine innenseitig umlaufende Wulst (58) aufweist, die in eine korrespondierende umlaufende Außennut (60) in der Fixierschürze (56) eingreift.

6. Wirkstoffampulle (2) nach einem der Ansprüche 1 bis 5, deren Verschlusskörper (50) aus TPE hergestellt ist.

7. Wirkstoffampulle (2) nach einem der Ansprüche 1 bis 6 mit einem innerhalb des Hohlkörpers (16) verschiebbaren Stopfen (62), hergestellt aus TPE.

8. Medizinische Spritze (1) mit einer Wirkstoffampulle (2) nach einem der Ansprüche 1 bis 7, an der endseitig einerseits ein eine Hohlnadel (14) tragender Nadelkopf (6) und andererseits ein Griffstück (10) anbringbar sind.

9. Medizinische Spritze (1) nach Anspruch 8, deren Nadelkopf (6) einen Grundkörper (20) aufweist, an dem zur Bildung einer Nadelschutzhülle (26) über ein Gelenk (28) ein Schutzkörper (30) schwenkbar angebracht ist, der in seiner Länge die freie Länge der Hohlnadel (14) übertrifft und diese somit endseitig überragt.

10. Verfahren zur Herstellung einer Wirkstoffampulle (2) nach einem der Ansprüche 1 bis 7, bei dem der Hohlkörper (16) und der in diesem vorgesehene Verschlusskörper (50) in einem gemeinsamen Arbeitsschritt in einem 2K-Spritzgussverfahren hergestellt werden.

11. Verfahren zum Befüllen einer Wirkstoffampulle (2) nach einem der Ansprüche 1 bis 7 mit Wirkstoff, bei dem der Hohlkörper (16) mit seiner Längsrichtung in eine vertikale Position gebracht wird, bei der das mit dem Verschlusskörper (50) versehene Ende (4) abwärts ausgerichtet ist und der Hohlkörper (16) anschließend über sein offenes Ende (8) befüllt wird.
